# EUROPEAN PATENT SPECIFICATION

(11) **EP 2 818 197 B1**
(45) Date of publication and mention of the grant of the patent: **27.02.2019**
(21) Application number: 14175086.9
(22) Date of filing: 30.06.2014
(51) Int. Cl.: A61M 25/00, A61M 1/36, A61M 5/142, A61M 5/14

(54) **Catheter**
Katheter
Cathéter

(30) Priority: 28.06.2013 IT BO20130338
(43) Date of publication of application: 31.12.2014
(73) Proprietor: Longo, Raffaele, 40124 Bologna (IT)
(72) Inventor: Longo, Raffaele, 40124 BOLOGNA (IT)
(74) Representative: Fuochi, Riccardo

(56) References cited:
- EP-A2- 0 426 319
- WO-A1-95/28188
- WO-A1-2010/054668
- WO-A2-02/05873
- US-A- 3 486 539
- US-A- 5 188 603
- US-A- 5 897 530

## Description

### TECHNICAL FIELD OF THE INVENTION

The present invention concerns a catheter for haemodialysis which does not have occlusions in the periods between one haemodialysis treatment and another. Other similar applications may be described as examples but do not form of the invention.

### STATE OF THE ART

There are different types of known catheters used for example for haemodialysis treatment in patients suffering from serious renal insufficiency, or even in other applications in hospitals.

For example, in the particular case of haemodialysis there are known central venous catheters generally comprising a tubular end part equipped with a distal end suitable for being inserted in a vein of the patient with or without tunneling, i.e. the subcutaneous insertion of the catheter for a predetermined part before access to the vessel. The end part is associated with one or more proximal cannulas suitable for connection to respective blood lines of machines for haemodialysis; the cannulas are affected by respective lumens that pass through the catheter for their entire length, including the end part.

According to the types, catheters for haemodialysis, once implanted in the patient, can remain inserted on site for the time necessary for the different prescribed haemodialysis treatments, and thus even for many months, before the normal removal and/or replacement.

Once the haemodialysis treatment has ended, the proximal cannula or cannulas are disconnected from the blood lines of the machine for haemodialysis, after which comes the accurate washing of the catheter with physiological solution, then the filling thereof with anti-coagulant fluid - like for example solutions of heparin, sodium citrate, physiological solution, and yet others - and the closing of the lumens with respective caps suitable for the purpose inserted at the ends of the cannulas.

The treatments can be carried out for many days in a week, or even daily.

The insertion of the anti-coagulant fluid obviously has the task of avoiding the formation of fibrin or of coagulated blood, or other similar phenomena, inside the catheter in the periods of time between one haemodialysis treatment and another, to try to prevent occlusion.

Before proceeding to the subsequent haemodialysis treatment, specialized nursing staff takes care of removing the caps of the cannulas, drawing the anti-coagulant fluid - and in part blood - out from the catheter, washing the latter carefully with physiological solution and then connecting the cannulas to the blood lines of the machine for haemodialysis.

However, unfortunately it is not uncommon, when treatment starts, for the catheter to still be occluded, not allowing the passage of blood from the patient to the machine and actually preventing the haemodialysis: this happens because the simple filling of anti-coagulant fluid of the catheter by itself does not always avoid the formation of occlusions for example of fibrin and/or coagulated blood.

This results in the obvious drawbacks, first of all for the patient, and also for the specialized nursing staff.

Indeed, in attempting to remove the occlusion, the nursing staff is forced to perform further and careful washing of the catheter, or even to fill it with solutions of fibrinolytic drugs - like for example urokinase, and others - with obvious discomfort for the patient, a delay to the start of the treatment and use of drugs that may even be expensive.

In some cases it may not be possible to eliminate the occlusion, and therefore it becomes necessary to remove the catheter before time and to replace it, with consequent and substantial discomfort especially for the patient.

Patent n. US 5,188,603 discloses a fluid infusion delivery system comprising a primary pump for delivering a medicament into an implanted catheter in a vein, and a second pump for delivering saline solution into the catheter in order to keep the vein open upon completion of the delivery of the medicament of the vein.

Patent application n. WO 2010/054668 discloses a dialysis catheter with a reservoir; after dialysis treatment the reservoir is filled with saline or flush solution or medication to create a positive pressure and a continuous flow inside the catheter lumina between dialysis sessions to prevent blood flowing back into catheter lumina and thrombosis formation.

### PURPOSES OF THE INVENTION

The technical task of the present invention is to improve the state of the art.

In such a technical task, a purpose of the present invention is to devise a catheter that, in the case of application in haemodialysis, prevents the formation of occlusions inside it during the periods of time between one haemodialysis treatment and another, maintaining the optimal openness of the lumens.

Yet another purpose of the present invention is to make a catheter that is simple to use and quick to use and maintain.

This task and these purposes are accomplished by the catheter for haemodialysis according to the attached claim 1.

The catheter is of the type comprising at least one tubular end part equipped with at least one distal end suitable for being inserted in a vein of the patient, a first proximal cannula and a second proximal cannula, associated with said end part, for the connection to blood lines of machines for haemodialysis or similar.

The first and the second cannula are respectively provided with a first lumen and by a second lumen for the passage of blood and medical fluids.

The catheter also comprises pressurised release means arranged in communication with the first lumen and/or the second lumen for the controlled dispensing of an anti-coagulant fluid in the periods between one treatment and another, so as to prevent the formation of occlusions in said catheter.

The pressurised release means comprise an elastomeric pump contained inside a rigid casing; the casing can be removably associated, at a first opening for dispensing the anti-coagulant fluid, with the first cannula and/or with the second cannula through a first coupling of the quick-acting type and/or a second coupling of the quick-acting type.

According to the invention, the casing comprises a second opening for filling the elastomeric pump with anti-coagulant fluid, at which there is a respective closing cap of the quick-acting type.

The dependent claims refer to preferred and advantageous embodiments of the invention.

### BRIEF DESCRIPTION OF THE DRAWINGS.

The characteristics of the invention will become clearer to any man skilled in the art from the following description and from the attached tables of drawings, given as a non-limiting example, in which:
figure 1 is a perspective view of a catheter according to the present invention;
figure 2 is a detailed and partially sectioned side view of the pressurised release means of the catheter;
figure 3 is a perspective view of another embodiment of the catheter according to the invention;
figure 4 is a perspective view of another embodiment of the catheter according to the invention;
figure 5 is a detailed and partially sectioned side view of the pressurised release means of the catheter of figure 4;
figure 6 is a perspective view of another embodiment of the catheter according to the invention;
figure 7 is a perspective view of a further embodiment of the catheter according to the present invention.

### EMBODIMENTS OF THE INVENTION.

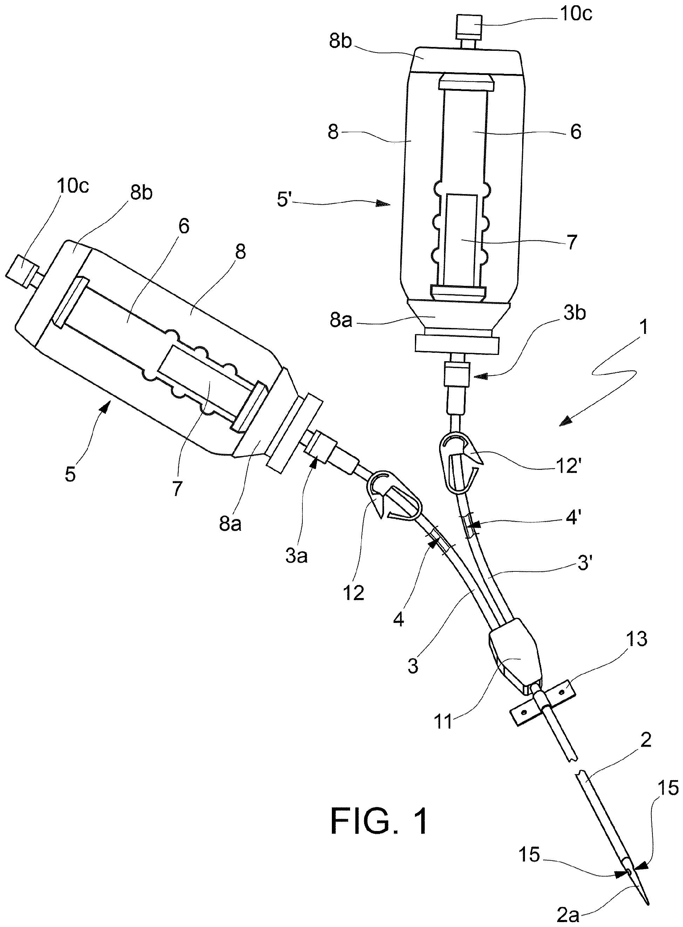

With reference to the attached figure 1, a catheter according to the present invention is wholly indicated with 1.

In the described embodiment the catheter 1 is for haemodialysis treatment, but it could, more generally, also be applied to other types of treatment, without such application forming part of the invention. The catheter 1 is of the type comprising at least one tubular end part 2.

The tubular end part 2 is equipped with at least one distal end 2a, suitable for being inserted in a vein of the patient.

Moreover, the catheter 1 comprises at least one proximal cannula 3,3' associated with the end part 2. The at least one proximal cannula 3,3' is suitable for connection to blood lines of machines for haemodialysis, or of other similar machinery intended to carry out other types of treatment, without such application forming part of the invention. In the particular case of haemodialysis, such machines are of the *per sé* known type and do not constitute the object of the present invention, and therefore they will not be described any further.

The end part 2 and the at least one proximal cannula 3,3' are affected by at least one lumen 4,4' for the passage of blood and medical fluids.

In greater detail, in all of the embodiments described hereafter, the catheter 1 comprises a first cannula 3 and a second cannula 3', for connection to respective blood lines, for example for intake and delivery.

The cannulas 3,3', for example in the case of use in dialysis treatment, can also comprise respective differently coloured portions to distinguish the one conventionally called "arterial" - red portion - from which blood is normally drawn from the patient towards the machine, from the one called "venous" - blue portion - from which blood is normally returned from the machine to the patient.

The catheter comprises a joining element 11 that places the first cannula 3 and the second cannula 3' in communication with the end part 2.

The first cannula 3 and the second cannula 3' are respectively affected by a first lumen 4 and by a second lumen 4', and the two lumens 4,4' pass through the end part 2.

The first cannula 3 and the second cannula 3' comprise, at the respective free ends, respective couplings of the quick-acting type 3a,3b.

In greater detail, the first cannula 3 comprises a first coupling of the quick-acting type 3a, whereas the second cannula 3' comprises a second coupling of the quick-acting type 3b.

The first coupling of the quick-acting type 3a and the second coupling of the quick-acting type 3b make it possible, for example, to respectively connect the first cannula 3 and the second cannula 3' to blood lines of machines for haemodialysis, or of other similar machinery.

The first coupling of the quick-acting type 3a and the second coupling of the quick-acting type 3b can, for example, be of the type using screw, bayonet or similar coupling.

According to an aspect of the present invention, the catheter 1 comprises pressurised release means 5,5', arranged in communication with at least one from the first lumen 4 and the second lumen 4', for the controlled dispensing of an anti-coagulant fluid in the periods between one treatment and another, for example between one dialysis treatment and another.

As will become clearer hereafter, this solution prevents the formation of occlusions in the catheter 1 itself in the aforementioned periods between one treatment and another, for example between one dialysis treatment and another.

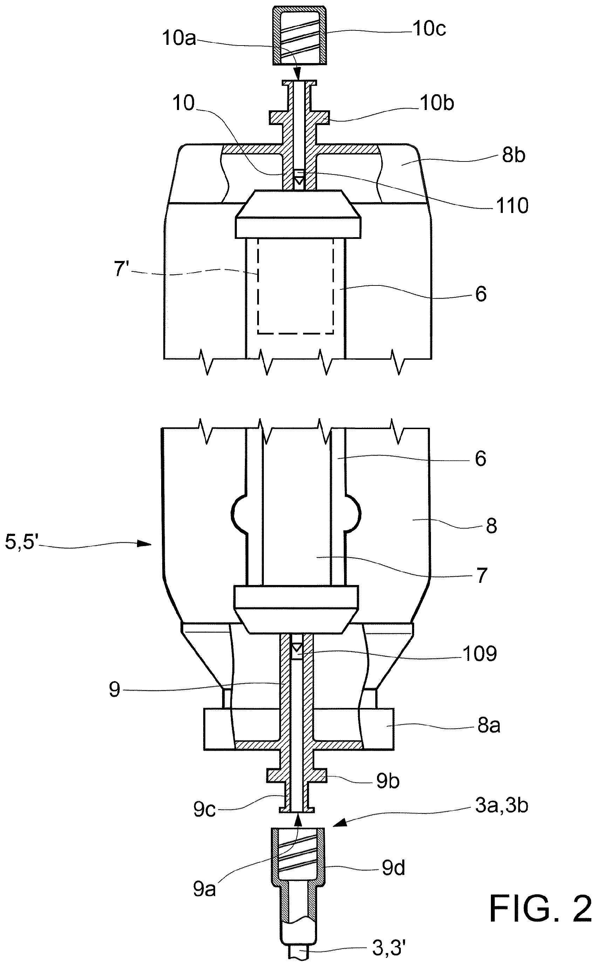

In greater detail, and with particular reference to the embodiment illustrated in figures 1,2, the catheter according to the invention comprises first pressurised release means 5 and second pressurised release means 5' respectively arranged in communication with the first lumen 4 and with the second lumen 4' in a removable manner.

This makes it possible to keep the release means 5,5' connected to the lumens 4,4' in the periods between one treatment and another, and to remove them typically when the treatment needs to begin.

In the rest of the present description we will assume that the first release means 5 and the second release means 5' are constructively identical to one another, or at least that the differences between them are insubstantial.

In any case, it should be specified that in some embodiments of the invention the first release means 5 and the second release means 5' could be constructively different from one another in some aspects.

The release means 5,5' each comprise an elastomeric pump 6.

The latter consists of a tank made of elastic material, of predetermined capacity.

The elastomeric pump 6 is, for example, inserted on a tubular support 7.

The release means 5,5' also each comprise a rigid casing 8, inside which the elastomeric pump 6 is housed.

The casing 8 mainly has the function of protecting the elastomeric pump 6 from possible accidental mechanical actions that could influence its operation.

Such protective action of the outer casing 8 must be particularly effective and safe since the catheter 1 according to the invention must be permanently worn by the patient in all the periods between one treatment and another, in particular between one dialysis treatment and another.

The casing 8 can for example by cylindrical or substantially cylindrical in shape.

Alternatively, the casing 8 can have any other shape suitable for the application.

The cylindrical shape makes it possible to reduce the discomfort of the patient when wearing the catheter.

The casing 8 comprises a first end 8a and an opposite second end 8b.

In particular, at the first end 8a the removable connection is made with the first cannula 3 or with the second cannula 3'.

The tubular support 7 for the elastomeric pump 6 is fixedly connected to the first end 8a.

In the particular embodiment of the invention illustrated in figures 1,2, the elastomeric pump 6 is of the type open at both ends.

Regarding this, a second tubular support 7' - illustrated with a broken line in figure 2 - can be foreseen at the second end 8b of the casing 8.

Other aspects of the invention will become clearer by observing figure 2, in which the first release means 5 are illustrated in detail.

The casing 8 comprises a first duct 9.

The first duct 9 is foreseen at the first end 8a of the casing 8.

The first duct 9 communicates with the elastomeric pump 6.

The first duct 9 defines a first opening 9a for dispensing the fluid contained in the elastomeric pump 6 through the first cannula 3, or equally through the second cannula 3'.

In the embodiment of the invention illustrated in figures 1,2, the first duct 9 extends outside the casing 8 with a first appendage 9b that comprises, in fact, the first member 9c of the first quick connection element 3a, or equally of the second quick connection element 3b.

The second member 9d of the first quick connection element 3a, or equally of the second quick connection element 3b, is directly associated with the first cannula 3, or respectively with the second cannula 3'. The casing 8 comprises a second opening 10a.

The second opening 10a communicates with the elastomeric pump 6.

The casing 8 comprises, at its second end 8b, a second duct 10.

The second duct 10 defines the second opening 10a.

The second opening 10a is suitable for filling the cavity of the elastomeric pump 6 with an anti-coagulant fluid.

In the embodiment of the invention illustrated in figures 1,2, the second duct 10 extends outside the casing 8 with a second appendage 10b.

The second duct 10 is associated with a closing cap 10c.

In particular, the closing cap 10c is removably coupled with the second appendage 10b.

The closing cap 10c is of the quick-acting type.

The closing cap 10c makes it possible to selectively access, at the user's discretion, the cavity of the elastomeric pump 6, as described better hereafter.

The coupling between the closing cap 10c and the second appendage 10b can be of the screw, bayonet or similar type, or in any case a quick coupling that allows removal and engagement with a few simple gestures.

Along the first duct 9 there is a first unidirectional valve 109.

The first unidirectional valve 109 allows the anti-coagulant fluid to flow only in the sense exiting from the cavity of the elastomeric pump 6.

Along the second duct 10 there is a second unidirectional valve 110.

The second unidirectional valve 110 allows the anti-coagulant fluid to flow only in the sense of entry into the cavity of the elastomeric pump 6.

Each of the cannulas 3,3' of the catheter 1 comprises, or can comprise, a respective first safety closing clip 12 and second safety closing clip 12'.

The catheter 1 comprises, or in any case can comprise, means 13 for fixing to the body of the patient.

For example, such fastening members 13 can consist of peripheral tabs distributed over the end part 2, or over the joining element 11.

The distal end 2a comprises end holes 15 that, in the case of dialysis treatment, allow blood to be drawn and returned.

The catheter 1 is made from biocompatible material, of the type that is *per sé* known in this type of applications.

The way of using the catheter 1, according to the invention is, according to what is described, totally intuitive.

The end part 2 is implanted to the patient with the distal end 2a inserted in a vein - with or without tunneling - and possibly stabilized at the patient himself through the aforementioned fastening means 13. The two lumens 4,4' intercept from the distal end 2a - through the end holes 15 - the vessel of the patient, one for drawing and the other for returning blood.

In order to start the treatment through the machine for haemodialysis, or through other similar machinery used in hospitals that does not form part of the invention, it is foreseen to connect the blood line for drawing blood to the first cannula 3, and the blood line for returning blood to the second cannula 3' - or vice-versa - so as to allow extracorporeal circulation and dialysis, or another treatment, in the machine of blood.

Once the treatment has ended, the catheter 1 is washed carefully with physiological solution, then filled with anti-coagulant fluid.

Such anti-coagulant fluid can consist of, or can comprise, for example solutions of heparin, sodium citrate, physiological solution, and others.

At this point - possibly after closing the first clip 12 and the second clip 12' - the first pressurised release means 5 are connected to the first cannula 3 through the first quick connection element 3a, whereas the second release means 5' are connected to the second cannula 3' through the second coupling of the quick-acting type 3b, thus obtaining the configuration of figure 1.

It is now possible to fill the elastomeric pumps 6 of the first and second pressurised release means 5,5' - or even of just one of them - with anti-coagulant fluid of the type defined earlier, by disconnecting the caps 10c of the respective casings 8.

The elastomeric pumps 6 are thus filled like a ball and, due to their elastic characteristics, then tend to return slowly to the original dimensions, thus dispensing the anti-coagulant fluid through the first cannula 3 and the second cannula 3'.

In this way, during the period before the next treatment, the elastomeric pumps 6 dispense a predetermined flow of anti-coagulant fluid under pressure in the lumens 4,4', which will prevent any formation of occlusions, such as coagulated blood, fibrin, or other similar phenomena, especially at the holes 15.

When it is necessary to carry out a treatment again, it will be sufficient to disconnect the release means 5,5' through the respective couplings of the quick-acting type 3a,3b: the operator will thus find the patient's vessel, and the two lumens 4,4', already perfectly cleaned and open in order to be able to be connected to the blood lines of the dialysis machine or another other machinery, not forming part of the invention, after haematic flushing according to protocols and/or guidelines.

The elastomeric pumps 6 of the catheter 1 are suitably sized so as to dispense the right amount of fluid for the entire period before the next treatment.

For example, the duration of the dispensing must be at least 24 hours, or even 48 hours, uninterrupted.

In the particular case of use in patients that carry out dialysis, the catheter 1 according to the invention can allow even just 0,1 ml/h of anti-coagulant fluid to be dispensed.

This appreciable result, which is particularly significant above all for patients who undergo the dialysis treatment, is obtained using elastomeric pumps 6 of particularly small dimensions, suitably devised for this application and not currently available on the market.

The result is also substantially important in that - like in experimental readings - it is possible to use anti-coagulant fluid consisting of just physiological solution.

In other words, just the flow of liquid ensured by the pressurised release means 5,5' allows - with a truly very low flow rate if liquid - the lumens 4,4' and the vessel of the patient to be kept open without the anti-coagulant effect being determined pharmacologically. Then considering the fact that the catheter 1, with the relative pressurised release means 5,5', must be worn by the patient possibly during the entire period of time between one treatment and another, the dimensions and the weights of the aforementioned release means 5,5' must be particularly low.

This is obtained thanks to the particular opposite arrangement of the first opening 9a for dispensing and of the second opening 10a for filling, which makes it possible to contain the transversal bulk of the casing 8 to be contained.

The simultaneous presence of two pressurised release means 5,5' in any case ensures continuity of dispensing of the anti-coagulant fluid even in the case in which one of the elastomeric pumps 6 has run out.

In this way, the discomfort felt by the patient due to the need to always wear the catheter in the period between one treatment and another, is reduced thanks to the fact of being able to use two release means 5,5', which can both be made with very small dimensions, rather than just one of greater dimensions, for the same fluid dispensed in the same time period.

Each of them can thus be moved and positioned as desired so as to cause the least possible discomfort.

It should also be noted that the pressurised release means 5,5' are not of the disposable type, but as stated the respective elastomeric pumps 6 can be conveniently filled as needed, possibly also without disconnecting the aforementioned release means 5,5' from the respective cannulas 3,3', thanks to the presence of the caps 10c.

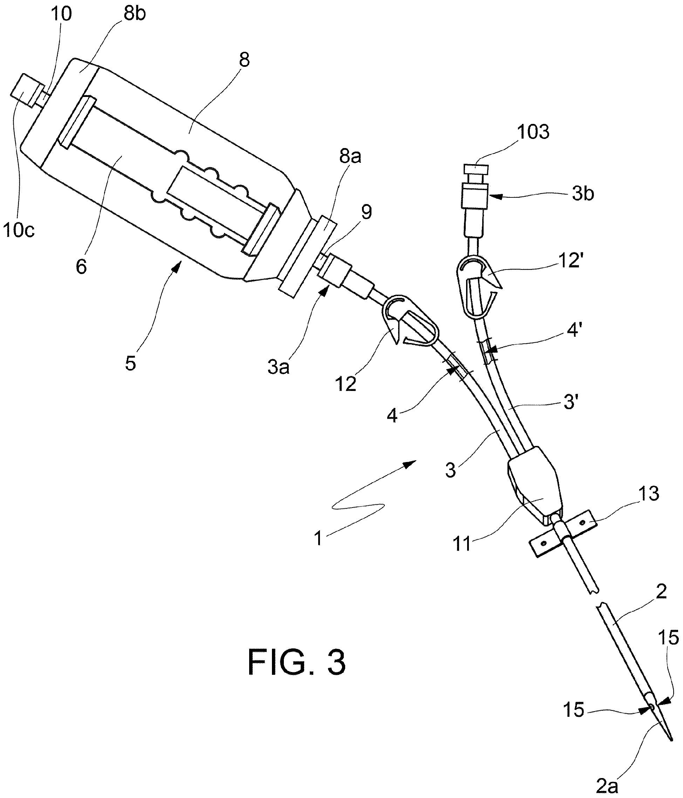

Figure 3 illustrates another embodiment of the catheter 1 according to the present invention.

It should be specified that in the aforementioned figure 3, and in the following description, the parts corresponding to those of the previous embodiment are indicated with the same reference numerals.

This embodiment is constructively simplified with respect to that of figures 1,2.

In this embodiment, the catheter 1 comprises just the first pressurised release means 5 associated with the first cannula 3.

The first pressurised release means 5 are identical to those described for the previous embodiment.

The second coupling of the quick-acting type 3b, not being associated in this case with pressurised release means, is instead associated with a closing element 103 of the quick-acting, screw, bayonet or similar type. This embodiment should be considered particularly advantageous in the case in which the requirements of containing the dimensions and the weight of the pressurised release means 5 are particularly important, for example in use on patients that are very old or disabled; of course, given the presence of a single elastomeric pump 6, the continuity of dispensing is ensured for a shorter period of time with respect to the previous embodiment.

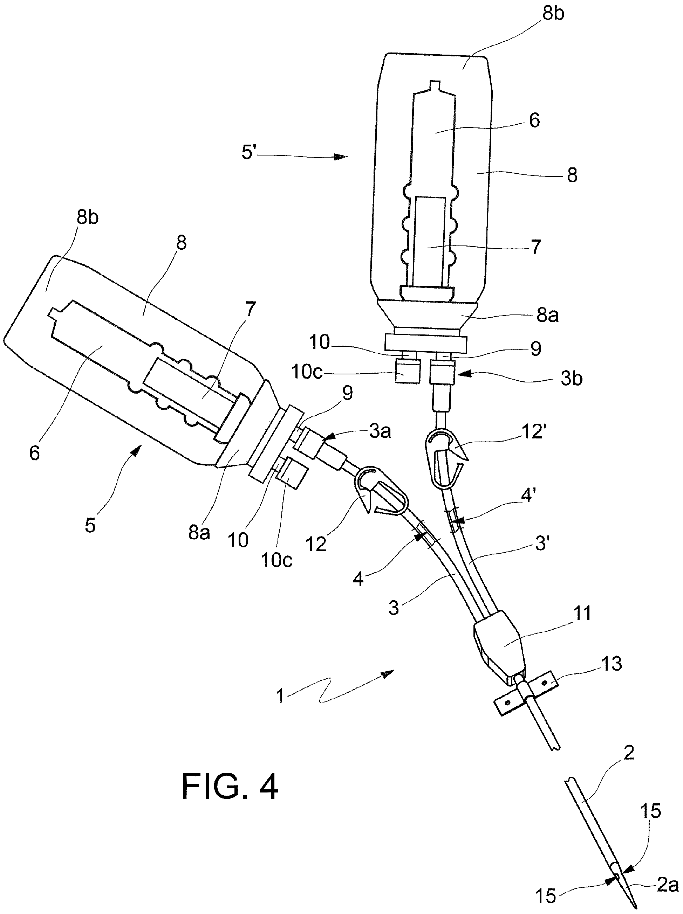

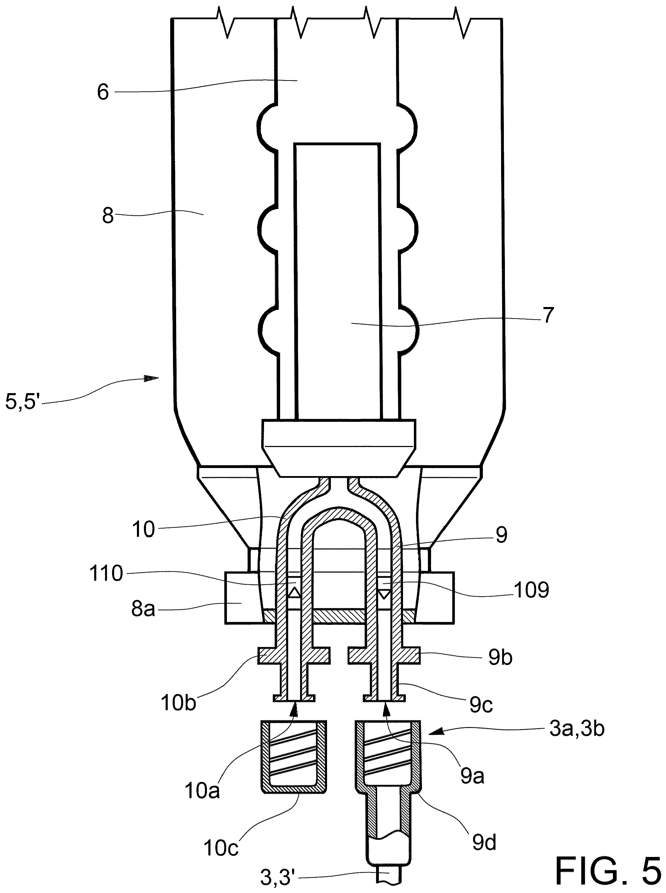

Yet another embodiment of the catheter according to the invention is illustrated in figures 4,5.

It should be specified that in the aforementioned figures 4,5, and in the following description, the parts corresponding to those of the previous embodiments are indicated with the same reference numerals.

This embodiment differs from the previous ones mainly for the configuration of the pressurised release means 5,5' .

Indeed, in this embodiment, in each of the first and second pressurised release means 5,5', the first opening 9a and the second opening 10a respectively for dispensing and filling the elastomeric pump 6 are both located at the first end 8a of the casing 8, as illustrated in particular in the detail of figure 5. One of the main advantages connected to the use of this solution is the greater ease and practicality of filling the cavity of the elastomeric pump 6 of each of the release means 5,5' with the anti-coagulant fluid, since the casing 8 possesses a support base at the second end 8b.

Moreover, this solution makes it possible to use an elastomeric pump 6 that is open on just one side, and therefore of the type already available on the market. In detail, in this embodiment the first duct 9, for dispensing, and the second duct 10, for filling, are foreseen at the first end 8a of the casing 8.

The first duct 9 and the second duct 10 can communicate, like in the solution of figure 5, defining a sort of single U-shaped duct, or alternatively they can be completely separate from one another.

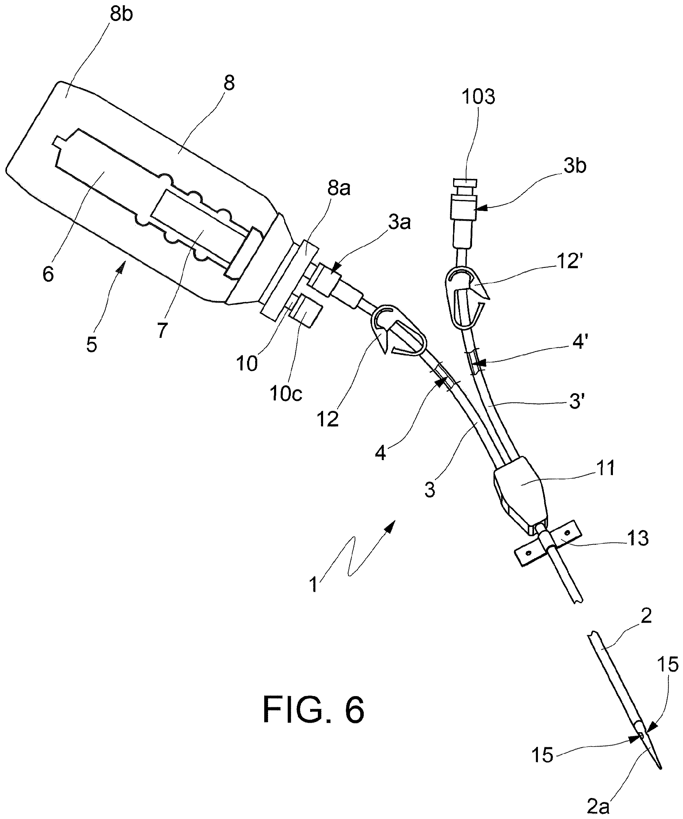

Yet another embodiment of the catheter 1 according to the invention is illustrated in figure 6.

In the aforementioned figure 6, and in the following description, the parts corresponding to those of the previous embodiment are indicated with the same reference numerals.

This embodiment is constructively simplified with respect to the one of figures 4,5.

In this embodiment, the catheter 1 comprises just the first pressurised release means 5, associated with the first cannula 3.

The first pressurised release means 5 are identical to those described for the previous embodiment.

The second coupling of the quick-acting type 3b, not being associated in this case with pressurised release means, is instead associated with a closing element 103 of the quick-acting, screw, bayonet or similar type.

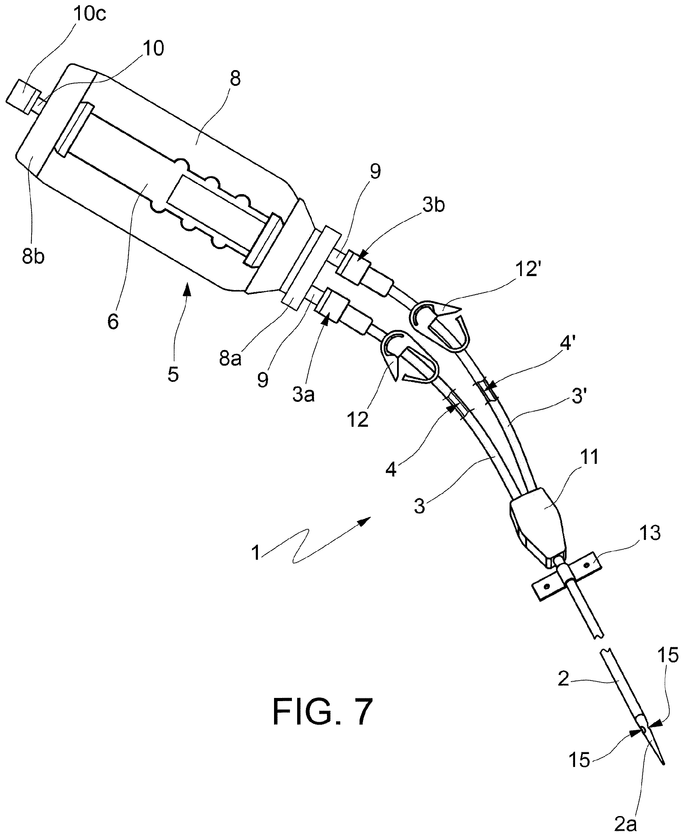

This embodiment should also be considered particularly advantageous in the case in which the requirements of containing the dimensions and the weight of the pressurised release means 5 are particularly important, for example in use on very old or disabled patients. Figure 7 illustrates yet another embodiment of the catheter 1 according to the present invention.

In the aforementioned figure 7, and in the following description, the parts corresponding to those of the previous embodiment are indicated with the same reference numerals.

In this embodiment, a single first pressurised release means 5 is used to dispense anti-coagulant fluid both along the first cannula 3 and along the second cannula 3'.

In detail, a single elastomeric pump 6 is placed in communication both with the first cannula 3 and with the second cannula 3'.

The casing 8 thus comprises, at its first end 8a, two parallel first ducts 9 for dispensing anti-coagulant fluid, respectively associated with the first cannula 3 and with the second cannula 3' through the first coupling of the quick-acting type 3a and the second coupling of the quick-acting type 3b.

Moreover, like in the embodiments of figures 4-6, it is foreseen for there to be a second duct 10, with relative cap 10c, for filling the elastomeric pump 6. The second duct 10 is opposite the first ducts 9, i.e. it is foreseen at the second end 8b of the casing 8.

In other embodiments, the second duct 10 could also be foreseen at the first end 8a of the casing 8.

The first ducts 9 for dispensing the fluid can be rigid or, more suitably, flexible to facilitate the quick connection to the first cannula 3 and to the second cannula 3'.

This solution is particularly advantageous in the case in which it is wished to reduce to the minimum the bulk and the weight of the pressurised release means 5, but so that, at the same time, dispensing of anti-coagulant fluid is maintained along both of the lumens 4,4' of the catheter: it is thus avoided, with respect to the solutions of figures 3,6, that possible coagulations deposit at the lumen 4' not directly affected by the flow of the fluid.

The invention, thus conceived, makes it possible to obtain important technical advantages.

An important technical advantage consists of the fact that the catheter 1 according to the invention, thanks to the controlled dispensing under pressure of anti-coagulant fluid through the release means 5,5', prevents the formation of any occlusion inside it during the periods of time between one treatment and another, maintaining the optimal openness of the lumens 4,4'.

The catheter 1, thanks to the provision of the release means 5,5', can thus remain normally implanted in the patient for the entire duration foreseen, above all avoiding the substantial discomfort that the patient himself would inevitably be subjected to following the non-rare occlusions in known catheters.

Moreover, the catheter 1 described simplifies the operations of the specialized nursing staff, also reducing the time and costs relating to the no longer necessary operations of removal of occlusions, use of further drugs, which may even be expensive, or replacement of the occluded catheter with another.

It should also be noted that the dispensing of the anti-coagulant fluid, which keeps the lumens 4 and the respective openings 15 open, is carried out with such small amounts as to be absolutely innocuous for the patient even in the case in which they were to reach the blood circulation.

Another important advantage consists of the fact that the openness of the lumens 4,4', and therefore of the vessel of the patient, can be effectively obtained using exclusively physiological solution, and no longer anti-coagulant drugs.

There is thus also a very substantial reduction in the costs associated with the treatments undergone by each patient.

The presence, in the casing, of a second opening suitably dedicated to filling the elastomeric pump makes it possible to reduce the healthcare waste, as well as the number of interventions on the patient to maintain the openness of the catheter and of the vessel.

Moreover, the presence of a quick-fastening closing cap makes it possible to use the same pressurised release means many times, without having to replace the casing and the relative elastomeric pump each time.

The catheter according to the invention has universal use, and does not need dedicated supports.

The catheter according to the invention makes it possible to decisively reduce the management interventions of each peripheral or central vascular access, since it replaces all of the manoeuvres for maintaining openness.

By reducing the interventions, probable sources of infection are also reduced.

As well as a reduction in the use of anti-coagulant drugs inside the solutions used for maintaining openness, a reduction of the drugs used systemically is also obtained.

A high reduction of healthcare waste is also obtained. Lastly, it should be specified that the catheter according to the invention can also be of the disposable type.

It has thus been seen how the invention achieves the proposed purposes.

The present invention has been described according to preferred embodiments, but equivalent variants can be devised without departing from the scope of protection offered by the following claims.

## Claims

1. Catheter (1) for haemodialysis treatment, of the type comprising
at least one tubular end part (2) equipped with at least one distal end (2a) suitable for being inserted in a vein of the patient,
a first proximal cannula (3) and a second proximal cannula (3'), associated with said end part (2), for the connection to blood lines of machines for haemodialysis,
said first and second cannula (3,3') being respectively provided with a first lumen (4) and by a second lumen (4') for the passage of blood and medical fluids, pressurised release means (5,5';5) arranged in communication with said first lumen (4) and/or said second lumen (4') for the controlled dispensing of an anti-coagulant fluid in the periods between one treatment and another, so as to prevent the formation of occlusions in said catheter (1),
said pressurised release means (5,5';5) comprising an elastomeric pump (6), consisting of a tank made of elastic material of predetermined capacity contained inside a rigid casing (8), said casing (8) being able to be removably associated, at a first opening (9a) for dispensing the anti-coagulant fluid, with said prima cannula (3) and/or with said second cannula (3') through a first coupling of the quick-acting type (3a) and/or a second coupling of the quick-acting type (3b), said casing (8) comprising a second opening (10a) for filling said elastomeric pump (6) with anti-coagulant fluid, at which there is a respective closing cap of the quick-acting type (10c),
said casing (8) comprising at least one first duct (9), communicating with said elastomeric pump (6), defining said first opening (9a), and a second duct (10), communicating with said elastomeric pump (6), defining said second opening (10a),
wherein said first duct (9) extends outside said casing (8) with a first appendage (9b) that comprises the first member of said first coupling of the quick-acting type (3a) or of said second coupling of the quick-acting type (3b), the second member (9d) of the first coupling of the quick-acting type (3a), or of the second coupling of the quick-acting type (3b), being directly associated with said first cannula (3), or respectively with said second cannula (3').

2. Catheter according to claim 1, wherein said casing (6) comprises a first end (8a) and an opposite second end (8b), said first duct (9) being foreseen at said first end (8a), said second duct (10) being foreseen at said second end (8b).

3. Catheter, according to claim 1, wherein said casing (8) comprises a first end (8a) and an opposite second end (8b), said first duct (9) and said second duct (10) both being foreseen at said first end (8a).

4. Catheter according to one of the previous claims, wherein said first coupling of the quick-actin type (3a) and/or said second coupling of the quick-acting type (3b) are of the screw-coupling, bayonet, or similar type.

5. Catheter according to one of the previous claims, wherein said closing cap of the quick-acting type (10c) is associated with said second opening (10a) of said casing (8) through coupling of the screw, bayonet or similar type.

6. Catheter according to one of the previous claims, wherein said casing (8) comprises a first unidirectional valve (109) at said first opening (9a), suitable for allowing the flow of anti-coagulant fluid only in the sense exiting from said elastomeric pump (6).

7. Catheter according to one of the previous claims, wherein said casing (8) comprises a first unidirectional valve (110) at said second opening (10a), suitable for allowing the flow of anti-coagulant fluid only in the sense entering said elastomeric pump (6).

8. Catheter according to one of the previous claims, comprising a first pressurised release means (5) associated with said first cannula (3) through said first quick connection element (3a), and a closing element of the quick-acting type (103) associated with said second coupling of the quick-acting type (3b).

9. Catheter according to one of claims 2-8, wherein said casing (8) comprises two first openings (9a) for dispensing the anti-coagulant fluid, foreseen in two respective first ducts (9), respectively associated with said first coupling of the quick-acting type (3a) and with said second coupling of the quick-acting type (3b).

10. Catheter according to the previous claim, wherein said first openings (9a) are both foreseen at a first end (8a) of said casing (8).

11. Catheter according to claim 10 or 11, wherein said first ducts (9) are flexible.

12. Catheter according to one of claims 2-8, comprising first pressurised release means (5) and second pressurised release means (5') respectively associated with said first cannula (3) through a first coupling of the quick-acting type (3a), and with said second cannula (3') through a second coupling of the quick-acting type (3b).

13. Catheter according to one of the previous claims, wherein said first cannula (3) and said second cannula (3') respectively comprise a first safety clip (12) and a second safety clip (12').

14. Catheter according to one of the previous claims, comprising means (13) for fixing to the body of the patient,

## Patentansprüche

1. Katheter (1) zur Hämodialysebehandlung, des Typs umfassend
mindestens ein rohrförmiges Endteil (2), das mit mindestens einem distalen Ende (2a) ausgestattet ist, das zur Einführung in die Vene des Patienten geeignet ist,
eine erste proximale Kanüle (3) und eine zweite proximale Kanüle (3'), die mit dem besagten Endteil (2) verbunden sind, für die Verbindung mit Blutschläuchen von Maschinen für die Hämodialyse,
wobei die besagte erste und zweite Kanüle (3, 3') jeweils mit einem ersten Lumen (4) und mit einem zweiten Lumen (4') für den Durchfluss von Blut und medizinische Flüssigkeiten versehen sind, druckbeaufschlagte Freigabemittel (5, 5'; 5), die in Verbindung mit dem besagten ersten Lumen (4) und/oder dem besagten zweiten Lumen (4') angeordnet sind, um eine Antikoagulanzflüssigkeit in den Zeiträumen zwischen einer Behandlung und einer anderen kontrolliert abzugeben, sodass die Bildung von Okklusionen in dem besagten Katheter (1) verhindert wird,
wobei die besagten druckbeaufschlagten Freigabemittel (5, 5'; 5) eine Elastomerpumpe (6) umfassen, bestehend aus einem Tank aus elastischem Material mit vorgegebener Kapazität, der in einem starren Gehäuse (8) enthalten ist, wobei das besagte Gehäuse (8) abnehmbar verbunden werden kann, an einer ersten Öffnung (9a) für die Abgabe der Antikoagulanzflüssigkeit, mit der besagten ersten Kanüle (3) und/oder mit der besagten zweiten Kanüle (3') durch eine erste Kupplung schnellwirkenden Typs (3a) und/oder eine zweite Kupplung schnellwirkenden Typs (3b), wobei das besagte Gehäuse (8) eine zweite Öffnung (10a) zum Füllen der besagten Elastomerpumpe (6) mit Antikoagulanzflüssigkeit umfasst, an der sich eine jeweilige Verschlusskappe schnellwirkenden Typs (10c) befindet,
wobei das Gehäuse (8) mindestens einen ersten Kanal (9) umfasst,
der mit der besagten Elastomerpumpe (6) in Verbindung steht, der die besagte erste Öffnung (9a) definiert, und einen zweiten Kanal (10), der mit der besagten Elastomerpumpe (6) in Verbindung steht und die besagte zweite Öffnung (10a) definiert, worin
der besagte erste Kanal (9) sich außerhalb des besagten Gehäuses (8) mit einem ersten Fortsatz (9b) erstreckt, der das erste Element der besagten ersten Kupplung schnellwirkenden Typs (3a) oder der besagten zweiten Kupplung schnellwirkenden Typs (3b) umfasst, wobei das zweite Element (9d) der ersten Kupplung schnellwirkenden Typs (3a) oder der zweiten Kupplung schnellwirkenden Typs (3b) direkt mit der besagten ersten Kanüle (3) bzw. mit der besagten zweiten Kanüle (3') verbunden ist.

2. Katheter nach Anspruch 1, worin das besagte Gehäuse (8) ein erstes Ende (8a) und ein entgegengesetztes zweites Ende (8b) umfasst, wobei der besagte erste Kanal (9) an dem besagten ersten Ende (8a) vorgesehen ist, der besagte zweite Kanal (10) an dem besagten zweiten Ende (8b) vorgesehen ist.

3. Katheter nach Anspruch 1, worin das besagte Gehäuse (8) ein erstes Ende (8a) und ein entgegengesetztes zweites Ende (8b) umfasst, wobei der besagte erste Kanal (9) und der besagte zweite Kanal (10) beide an dem besagten ersten Ende (8a) vorgesehen sind.

4. Katheter nach einem der vorangegangenen Ansprüche, worin die besagte erste Kupplung schnellwirkenden Typs (3a) und/oder die besagte zweite Kupplung schnellwirkenden Typs (3b) vom Schraubkupplungstyp, Bajonetttyp oder ähnlichen Typs sind.

5. Katheter nach einem der vorangegangenen Ansprüche, worin die besagte Verschlusskappe schnellwirkenden Typs (10c) mit der besagten zweiten Öffnung (10a) des besagten Gehäuses (8) durch Kupplung der Schraube, des Bajonetts oder eines ähnlichen Typs verbunden ist.

6. Katheter nach einem der vorangegangenen Ansprüche, worin das besagte Gehäuse (8) ein erstes unidirektionales Ventil (109) an der besagten ersten Öffnung (9a) umfasst, das geeignet ist, den Fluss der Antikoagulanzflüssigkeit nur in die aus der besagten Elastomerpumpe (6) austretende Richtung zu gestatten.

7. Katheter nach einem der vorangegangenen Ansprüche, worin das besagte Gehäuse (8) ein erstes unidirektionales Ventil (110) an der besagten zweiten Öffnung (10a) umfasst, das geeignet ist, den Fluss der Antikoagulanzflüssigkeit nur in die in die besagte Elastomerpumpe (6) eintretende Richtung zu gestatten.

8. Katheter nach einem der vorangegangenen Ansprüche, umfassend ein erstes druckbeaufschlagtes Freigabemittel (5), das mit der besagten ersten Kanüle (3) durch das besagte erste Schnellverbindungselement (3a) verbunden ist, und ein Verschlusselement schnellwirkenden Typs (103), das mit der besagten zweiten Kupplung schnellwirkenden Typs (3b) verbunden ist.

9. Katheter nach einem der Ansprüche 2-8, worin das besagte Gehäuse (8) zwei erste Öffnungen (9a) zur Abgabe der Antikoagulanzflüssigkeit umfasst, die in zwei jeweiligen ersten Kanälen (9) vorgesehen sind, die jeweils mit der besagten ersten Kupplung schnellwirkenden Typs (3a) und der besagten zweiten Kupplung schnellwirkenden Typs (3b) verbunden sind.

10. Katheter nach dem vorangehenden Anspruch, worin die besagten ersten Öffnungen (9a) beide an einem ersten Ende (8a) des besagten Gehäuses (8) vorgesehen sind.

11. Katheter nach Anspruch 10 oder 11, worin die besagten ersten Kanäle (9) flexibel sind.

12. Katheter nach einem der Ansprüche 2-8, umfassend erste druckbeaufschlagte Freigabemittel (5) und zweite druckbeaufschlagte Freigabemittel (5'), die jeweils mit der besagten ersten Kanüle (3) durch eine erste Kupplung schnellwirkenden Typs (3a) und mit der besagten zweiten Kanüle (3') durch eine zweite Kupplung schnellwirkenden Typs (3b) verbunden sind.

13. Katheter nach einem der vorangegangenen Ansprüche, worin die besagte erste Kanüle (3) und die besagte zweite Kanüle (3') jeweils eine erste Sicherheitsklammer (12) und eine zweite Sicherheitsklammer (12') umfassen.

14. Katheter nach einem der vorangegangenen Ansprüche, umfassend Mittel (13) zur Befestigung am Körper des Patienten.

## Revendications

1. Cathéter (1) pour le traitement par hémodialyse, du type comprenant :
au moins une partie d'extrémité tubulaire (2) dotée d'au moins une extrémité distale (2a) adaptée pour être insérée dans une veine du patient,
une première canule proximale (3) et une deuxième canule proximale (3'), associée à ladite partie d'extrémité (2), pour le raccordement aux tuyaux de transport de sang des machines pour hémodialyse,
ladite première et ladite deuxième canule (3, 3') étant respectivement dotées d'une première lumen (4) et d'une deuxième lumen (4') pour le passage de sang et de fluides médicaux,
des moyens de libération pressurisés (5, 5' ; 5) disposés en communication avec ladite première lumen (4) et/ou ladite deuxième lumen(4') pour la distribution contrôlée d'un fluide anticoagulant dans les périodes entre un traitement et un autre, de façon à empêcher la formation d'occlusions dans ledit cathéter (1),
lesdits moyens de libération pressurisés (5, 5' ; 5) comprenant une pompe élastomère (6) composée d'un réservoir constitué de matière élastique de capacité prédéterminée contenu dans un boîtier rigide (8), ledit boîtier (8) pouvant être associé de manière amovible au niveau d'une première ouverture (9a) pour distribuer le fluide anticoagulant, avec ladite première canule (3) et/ou avec ladite deuxième canule (3') à travers un premier couplage de type rapide (3a) et/ou un deuxième couplage de type rapide (3b), ledit boîtier (8) comprenant une deuxième ouverture (10a) pour remplir ladite pompe élastomère (6) avec le fluide anticoagulant, sur laquelle se trouve un bouchon respectif de type rapide (10c),
ledit boîtier (8) comprenant au moins un premier conduit (9), en communication avec ladite pompe élastomère (6), définissant ladite première ouverture (9a), et un deuxième conduit (10), en communication avec ladite pompe élastomère (6), définissant ladite deuxième ouverture (10a), dans lequel
ledit premier conduit (9) s'étend à l'extérieur dudit boîtier (8) avec un premier appendice (9b) qui comprend le premier élément dudit premier couplage de type rapide (3a) ou dudit deuxième couplage de type rapide (3b), le deuxième élément (9d) du premier couplage de type rapide (3a), ou du deuxième couplage de type rapide (3b), étant associé directement à ladite première canule (3), ou respectivement avec ladite deuxième canule (3').

2. Cathéter selon la revendication 1, dans lequel ledit boîtier (8) comprend une première extrémité (8a) et une deuxième extrémité opposée (8b), ledit premier conduit (9) étant prévu à ladite première extrémité (8a), ledit deuxième conduit (10) étant prévu à ladite deuxième extrémité (8b).

3. Cathéter selon la revendication 1, dans lequel ledit boîtier (8) comprend une première extrémité (8a) et une deuxième extrémité opposée (8b), ledit premier conduit (9) et ledit deuxième conduit (10) étant tous les deux prévus à ladite première extrémité (8a).

4. Cathéter selon l'une des revendications précédentes, dans lequel ledit premier couplage de type rapide (3a) et/ou ledit deuxième couplage de type rapide (3b) sont du type à vis, à baïonnette, ou de type similaire.

5. Cathéter selon l'une des revendications précédentes, dans lequel ledit bouchon de type rapide (10c) est associé à ladite deuxième ouverture (10a) dudit boîtier (8) par le couplage à vis, à baïonnette ou de type similaire.

6. Cathéter selon l'une des revendications précédentes, dans lequel ledit boîtier (8) comprend un première valve unidirectionnelle (109) au niveau de ladite première ouverture (9a), adaptée pour permettre l'écoulement de fluide anticoagulant seulement dans le sens de sortie de ladite pompe élastomère (6).

7. Cathéter selon l'une des revendications précédentes, dans lequel ledit boîtier (8) comprend une première valve unidirectionnelle (110) au niveau de ladite deuxième ouverture (10a), adaptée pour permettre l'écoulement de fluide anticoagulant seulement dans le sens d'entrée de ladite pompe élastomère (6).

8. Cathéter selon l'une des revendications précédentes, comprenant des premiers moyens de libération pressurisés (5) associés à ladite première canule (3) à travers ledit premier élément de couplage rapide (3a), et un élément de fermeture de type rapide (103) associé audit deuxième couplage de type rapide (3b).

9. Cathéter selon l'une des revendications 2 à 8, dans lequel ledit boîtier (8) comprend deux premières ouvertures (9a) pour distribuer le fluide anticoagulant, prévues dans deux premiers conduits (9) respectifs, associés respectivement audit premier couplage de type rapide (3a) et audit deuxième couplage de type rapide (3b).

10. Cathéter selon la revendication précédente, dans lequel lesdites premières ouvertures (9a) sont toutes les deux prévues à une première extrémité (8a) dudit boîtier (8).

11. Cathéter selon la revendication 10 ou 11, dans lequel lesdits premiers conduits (9) sont flexibles.

12. Cathéter selon l'une des revendications 2 à 8, comprenant des premiers moyens de libération pressurisés (5) et des deuxièmes moyens de libération pressurisés (5') respectivement associés à ladite première canule (3) par un premier couplage de type rapide (3a), et à ladite deuxième canule (3') par un deuxième couplage de type rapide (3b).

13. Cathéter selon l'une des revendications précédentes, dans lequel ladite première canule (3) et ladite deuxième canule (3') comprennent respectivement une première bride de sécurité (12) et une deuxième bride de sécurité (12').

14. Cathéter selon l'une des revendications précédentes, comprenant des moyens (13) de fixation au corps du patient.
